# DEMANDE DE BREVET EUROPEEN

(11) **EP 2 078 708 A1**
(43) Date de publication de la demande: **15.07.2009**
(21) Numéro de dépôt: 09158967.1
(22) Date de dépôt: 16.12.1999
(51) Int. Cl.: C07C 17/20, C07C 17/38, C07C 19/08, C01B 7/19

(54) **Compositions azéotropiques, leur utilisation et procédés de synthèse d'un hydrofluoroalcane**

(30) Priorité: 18.12.1998 BE 9800910
(62) Demande divisionnaire de: 99967969.9
(71) Demandeur: SOLVAY (Société Anonyme), 1050 Bruxelles (BE)
(72) Inventeur: Lambert, Alain, 1320 Beauvechain (BE); Wilmet, Vincent, 1300 Wavre (BE)
(74) Mandataire: Mross, Stefan P.M.

(57) **Abrégé**

Des compositions azéotropiques ou pseudo-azéotropiques constituées essentiellement de 1,5 % molaire à 27,5 % molaire de 1,1,1,3,3-pentafluorobutane et de 72,5 % molaire à 98,5 % molaire de fluorure d'hydrogène et leur utilisation sont décrites.

## Description

La présente invention concerne un procédé de séparation d'un mélange comprenant un hydrofluoroalcane et du fluorure d'hydrogène, de même que des procédés de préparation d'un hydrofluoroalcane et des compositions azéotropiques.

Les hydrofluoroalcanes peuvent être préparés par réaction d'un précurseur chloré approprié avec du fluorure d'hydrogène, tel que décrit par exemple dans les demandes de brevets EP-A1-0699649 et WO-A1-97/15540 (au nom de SOLVAY) et dans la demande de brevet WO-A1-97/05089. Dans un tel procédé, à la sortie du réacteur, le mélange de produits réactionnels contient, outre l'hydrofluoroalcane désiré, du chlorure d'hydrogène provenant de l'élimination du ou des atomes de chlore du précurseur chloré de départ, du fluorure d'hydrogène, des intermédiaires chlorofluorés, généralement du précurseur chloré non transformé, éventuellement des diluants inertes, ainsi que divers sous-produits en faibles quantités. Etant donné que l'on travaille habituellement avec un excès de fluorure d'hydrogène par rapport au précurseur chloré, il subsiste le plus souvent du fluorure d'hydrogène non converti dans le mélange de produits réactionnels. Alors que la plupart des constituants du mélange de produits réactionnels peuvent être facilement séparés complètement par distillation, une séparation complète entre l'hydrofluoroalcane et le fluorure d'hydrogène est généralement très difficilement réalisable par distillation, ces composés formant en effet souvent des mélanges azéotropiques.

La demande de brevet WO-A1-97/05089 décrit un procédé de purification d'hydro(chloro)fluoroalcanes (en particulier, le 1,1,1,3,3-pentafluoropropane
ou HFC-245 fa) de mélanges azéotropiques avec le fluorure d'hydrogène par une technique de distillation azéotropique comprenant deux étapes de distillation successives à des températures et à des pressions différentes.

Cette technique de distillation azéotropique présente cependant les désavantages de requérir une grande différence de température ou de pression entre les deux colonnes de façon à disposer d'un potentiel de séparation suffisant (différence de composition entre l'azéotrope à basse pression/température et l'azéotrope à haute pression/température) et d'engendrer un important débit de circulation entre les deux colonnes.

La demande de brevet WO-A1-97/13719 divulgue un procédé de séparation et de récupération de fluorure d'hydrogène de ses mélanges (azéotropiques) avec, entre autres, des hydrofluoroalcanes contenant de 1 à 6 atomes de carbone (en particulier, le HFC-245fa). Le mélange est mis en contact avec une solution de fluorure de métal alcalin (en particulier, du fluorure de potassium ou de césium) et la phase organique est séparée de la phase contenant le fluorure d'hydrogène et le fluorure de métal alcalin.

Par ce procédé connu, on peut craindre la contamination de la phase organique par le fluorure de potassium ou de césium et le risque de décomposition des hydrofluoroalcanes que cette contamination pourrait entraîner. D'autre part, ces fluorures de métaux alcalins, et plus particulièrement le fluorure de césium, sont très coûteux.

La présente invention a pour objet de fournir un procédé pour la séparation d'un mélange comprenant au moins un hydrofluoroalcane, contenant de préférence de 3 à 6 atomes de carbone, et du fluorure d'hydrogène, qui ne présente pas les inconvénients des procédés précités.

L'invention concerne dès lors un procédé de séparation d'un mélange comprenant au moins un hydrofluoroalcane et du fluorure d'hydrogène, ci-après dénommé mélange hydrofluoroalcane/fluorure d'hydrogène, selon lequel on fait réagir le mélange hydrofluoroalcane/fluorure d'hydrogène avec au moins un composé organique capable de réagir avec du fluorure d'hydrogène.

La réaction du mélange hydrofluoroalcane/fluorure d'hydrogène avec au moins un composé organique capable de réagir avec du fluorure d'hydrogène permet de consommer au moins une partie du fluorure d'hydrogène. Le procédé de séparation selon l'invention fournit ainsi un mélange de produits réactionnels appauvri en fluorure d'hydrogène. Ceci présente de l'intérêt dans le cadre d'une synthèse d'hydrofluoroalcane, ledit mélange pouvant être utilisé notamment comme solvant d'extraction. Le mélange de produits réactionnels obtenu convient également bien comme produit de départ lorsqu'il est soumis à au moins une étape ultérieure de traitement destinée à récupérer l'hydrofluoroalcane. On peut ainsi obtenir de l'hydrofluoroalcane essentiellement exempt de fluorure d'hydrogène.

Un avantage particulier se présente lorsque l'hydrofluoroalcane est capable de former un azéotrope ou pseudo-azéotrope avec le fluorure d'hydrogène car il est possible de "casser" un tel azéotrope, c'est à dire que le procédé de séparation selon l'invention est capable de fournir un mélange dans lequel l'hydrofluoroalcane et le fluorure d'hydrogène sont présents dans des proportions différentes de celles pour lesquelles ils forment un azéotrope ou un pseudo-azéotrope.

Par hydrofluoroalcane, on entend désigner les composés hydrocarbonés répondant à la formule générale CₐH_{(2a+2)-b}F_{b} dans laquelle a = 1 à 6 et b = 1 à 2a+1. Les hydrofluoroalcanes qui contiennent de 3 à 6 atomes de carbone sont préférés. Les hydrofluoroalcanes qui contiennent de 3 à 4 atomes de carbone sont particulièrement préférés.

A titre d'exemples d'hydrofluoroalcanes séparables de mélanges avec le fluorure d'hydrogène par le procédé de séparation selon l'invention, on peut citer le 1,1,1,3,3-pentafluoropropane (HFC-245fa), le 1,1,2,2,3-pentafluoropropane (HFC-245ca), le 1,1,1,2,3-pentafluoropropane (HFC-245eb), le 1,1,1,3,3,3-hexafluoropropane (HFC-236fa), le 1,1,1,2,3,3-hexafluoropropane (HFC-236ea), le 1,1,1,2,3,3,3-heptafluoropropane (HFC-227ea), le 1,1,1,3,3-pentafluoro-2-méthylpropane (HFC-365mps), le 1,1,1,3,3-pentafluorobutane (HFC-365mfc), le 1,1,1,4,4,4-hexafluorobutane (HFC-356mff) et le 1,1,1,2,3,4,4,5,5,5-décafluoropentane (HFC-43-10mee). Parmi ces composés, le 1,1,1,3,3-pentafluoropropane (HFC-245fa) et le 1,1,1,3,3-pentafluorobutane (HFC-365mfc) sont particulièrement préférés. Le 1,1,1,3,3-pentafluorobutane (HFC-365mfc) est tout particulièrement préféré.

Les composés organiques utilisés dans le procédé de séparation selon l'invention sont capables de réagir avec du fluorure d'hydrogène. Des exemples de composé organique sont, entre autres, les alcanes linéaires ou ramifiés, de préférence chlorés et/ou bromés, éventuellement substitués comprenant de 1 à 10 atomes de carbone et les alcènes éventuellement substitués comprenant de 2 à 10 atomes de carbone. On met souvent en oeuvre un composé organique chloré ou chlorofluoré. Conviennent bien, par exemple, les oléfines halogénées telles que des oléfines chlorées, fluorées ou chlorofluorées, comme par exemple le chlorure de vinyle, le chlorure de vinylidène, le trichloroéthylène, le perchloroéthylène le fluorure de vinylidène, et le chlorotrifluoroéthylène ou des fluoropropènes tels que, par exemple, l'hexafluoropropène.

De préférence, le composé organique est un précurseur chloré ou chlorofluoré de l'hydrofluoroalcane.

Par précurseur chloré ou chlorofluoré de l'hydrofluoroalcane, on entend désigner des hydrochloroalcanes et des hydrochlorofluoroalcanes c'est-à-dire des composés hydrocarbonés respectivement chlorés et chlorofluorés contenant au moins un atome de chlore et au moins un atome d'hydrogène, le même nombre d'atomes de carbone que l'hydrofluoroalcane désiré et au moins un atome de fluor de moins que l'hydrofluoroalcane désiré. L'hydrofluoroalcane désiré peut être obtenu au départ d'au moins un précurseur chloré ou chlorofluoré de l'hydrofluoroalcane par une réaction de ce précurseur avec du fluorure d'hydrogène.

A titre d'exemples de précurseurs chlorés ou chlorofluorés d'hydrofluoroalcanes utilisables dans le procédé de séparation selon l'invention, on peut citer des hydrochloroalcanes tels le 1,1,1,3,3-pentachloropropane (HCC-240fa), le 1,1,2,2,3-pentachloropropane (HCC-240aa), le 1,1,1,2,3-pentachloropropane (HCC-240db), le 1,1,1,3,3,3-hexachloropropane (HCC-230fa), le 1,1,1,2,3,3-hexachloropropane (HCC-230da), le 1,1,1,2,3,3,3-heptachloropropane (HCC-220da), le 1,1,1,3,3-pentachloro-2-méthylpropane (HCC-360jns), le 1,1,1,3,3-pentachlorobutane (HCC-360jfa), le 1,1,1,4,4,4-hexachlorobutane (HCC-350jff) et le 1,1,1,2,3,4,4,5,5,5-décachloropentane (HCC-430jdd) et des hydrochlorofluoroalcanes tels le 1-fluoro-1,1,3,3-tétrachlorobutane (HCFC-361kfa), le 3-fluoro-1,1,1,3-tétrachlorobutane (HCFC-361jfb), le 1,1-difluoro-1,3,3-trichlorobutane (HCFC-3621fa), le 1,3-difluoro-1,1,3-trichlorobutane (HCFC-362kfb), le 3,3-difluoro-1,1,1-trichlorobutane (HCFC-362jfc), le 1,1-dichloro-1,3,3-trifluorobutane (HCFC-363kfc), le 1,3-dichloro-1,1,3-trifluorobutane (HCFC-3631fb), le 3,3-dichloro-1,1,1-trifluorobutane (HCFC-363mfa), le 1-chloro-1,1,3,3-tétrafluorobutane (HCFC-3641fc) et le 3-chloro-1,1,1,3-tétrafluorobutane (HCFC-364mfb).

Dans une variante, on entend désigner par précurseur chloré ou chlorofluoré de l'hydrofluoroalcane, des (hydro)chloroalcènes et des (hydro)chlorofluoroalcènes c'est-à-dire des composés carbonés respectivement chlorés et chlorofluorés contenant au moins un atome de chlore et éventuellement au moins un atome d'hydrogène, le même nombre d'atomes de carbone que l'hydrofluoroalcane désiré et au moins un atome de fluor de moins que l'hydrofluoroalcane désiré. Citons, par exemple des (hydro)chloro(fluoro)propènes tels que, par exemple, le 1-chloro-3,3,3-trifluoroprop-1-ène comme précurseur du 1,1,1,3,3-pentafluoropropane et les (hydro)chloro(fluoro)butènes tels que, par exemple les dichlorodifluorobutènes tels que notamment le 1,1-dichloro-1,3-difluorobut-2-ène et/ou les chlorotrifluorobutènes tels que notamment le 1-chloro-1,1,3-trifluorobut-2-ène comme précurseurs du 1,1,1,3,3-pentafluorobutane.

Dans le procédé de séparation selon l'invention, la réaction entre le mélange hydrofluoroalcane/fluorure d'hydrogène et le composé organique est de préférence effectuée en phase liquide.

Dans le procédé de séparation selon l'invention, la réaction entre le mélange hydrofluoroalcane/fluorure d'hydrogène et le composé organique peut être effectué en présence d'un catalyseur. Elle peut également être effectuée en l'absence de catalyseur.

Lorsqu'on effectue la réaction entre le mélange hydrofluoroalcane/fluorure d'hydrogène et le composé organique en présence d'un catalyseur, on peut mettre en oeuvre des catalyseurs pouvant favoriser la substitution d'un atome de chlore par un atome de fluor. Parmi les catalyseurs utilisables, on peut citer les dérivés des métaux choisis parmi les métaux des groupes IIIa, IVa et b, Va et b, VIb du Tableau Périodique des Eléments et leurs mélanges. On retient plus spécialement les dérivés du titane, du tantale, du molybdène, du bore, de l'étain et de l'antimoine. De préférence, on met en oeuvre des dérivés du titane ou de l'étain. Les dérivés du titane conviennent particulièrement bien. Comme dérivés des métaux, on peut citer les sels et plus particulièrement les halogénures. De préférence, on choisit parmi les chlorures, les fluorures et les chlorofluorures. Des catalyseurs particulièrement préférés dans le procédé de préparation de l'hydrofluoroalcane selon l'invention sont les chlorures, les fluorures et les chlorofluorures de titane et d'étain et leurs mélanges. Le tétrachlorure de titane et le tétrachlorure d'étain conviennent particulièrement bien.

Lorsque la réaction entre le mélange hydrofluoroalcane/fluorure d'hydrogène et le composé organique est effectuée en phase liquide, elle est de préférence effectuée en l'absence de catalyseur, ce qui permet de soutirer le milieu réactionnel en phase liquide et, le cas échéant, d'effectuer une ou plusieurs étapes de distillations aisées subséquentes.

Le rapport molaire entre le fluorure d'hydrogène et l'hydrofluoroalcane dans le mélange hydrofluoroalcane/fluorure d'hydrogène à séparer par le procédé de séparation selon l'invention est variable. Le fluorure d'hydrogène peut être en excès par rapport à l'hydrofluoroalcane dans le mélange hydrofluoroalcane/fluorure d'hydrogène à séparer par le procédé de séparation selon l'invention. Le procédé de séparation selon l'invention convient bien lorsque l'hydrofluoroalcane est capable de former un azéotrope ou un pseudo-azéotrope avec le fluorure d'hydrogène. De préférence, le procédé de séparation selon l'invention s'applique à un mélange hydrofluoroalcane/fluorure d'hydrogène de composition azéotropique ou proche de la composition azéotropique.

Le procédé de séparation selon l'invention trouve une utilisation avantageuse pour la séparation du mélange comprenant du 1,1,1,3,3-pentafluorobutane (HFC-365mfc) et du fluorure d'hydrogène.

A une pression de 3 bar, la composition du mélange azéotropique fluorure d'hydrogène/1,1,1,3,3-pentafluorobutane est d'environ 60/40 % en poids, soit un rapport molaire fluorure d'hydrogène/1,1,1,3,3-pentafluorobutane d'environ 11 mol/mol.

Dans une forme de réalisation préférée de l'invention, la séparation du mélange comprenant du 1,1,1,3,3-pentafluorobutane et du fluorure d'hydrogène est effectuée par la réaction du mélange 1,1,1,3,3-pentafluorobutane/fluorure d'hydrogène avec un précurseur du 1,1,1,3,3-pentafluorobutane, de préférence du 1,1,1,3,3-pentachlorobutane, en l'absence de catalyseur. Le 1,1,1,3,3-pentachlorobutane peut être obtenu par exemple par télomérisation de composés chlorés tels que par exemple du 2-chloropropène avec du tétrachlorure de carbone ou du chlorure de vinylidène avec du 1,1,1-trichloro éthane en présence de différents catalyseurs tels que notamment le fer pentacarbonyle ou des sels de cuivre en combinaison avec une amine.

Dans le procédé de séparation selon l'invention, on met en oeuvre le mélange hydrofluoroalcane/fluorure d'hydrogène et le composé organique dans des proportions telles que le rapport molaire entre le fluorure d'hydrogène et le composé organique est généralement d'au moins 0,5 mol/mol. De préférence, on travaille avec un rapport molaire entre le fluorure d'hydrogène et le composé organique d'au moins 1 mol/mol. De manière plus particulièrement préférée on travaille avec un rapport molaire entre le fluorure d'hydrogène et le composé organique d'au moins 3 mol/mol. De manière tout particulièrement préférée on travaille avec un rapport molaire entre le fluorure d'hydrogène et le composé organique d'au moins 5 mol/mol. Le rapport molaire entre le fluorure d'hydrogène et le composé organique mis en oeuvre ne dépasse en général pas 15 mol/mol. Il est préférable que ce rapport molaire ne dépasse pas 10 mol/mol.

La température à laquelle on effectue la réaction entre le mélange hydrofluoroalcane/fluorure d'hydrogène et le composé organique est généralement d'au moins 60°C. De préférence, la température est d'au moins 80°C. En général, la température ne dépasse pas 160°C. De préférence, elle ne dépasse pas 140°C.

La réaction entre le mélange hydrofluoroalcane/fluorure d'hydrogène et le composé organique dans le procédé de séparation selon l'invention est de préférence réalisée en phase liquide. Dans ce cas, la pression est choisie de manière à maintenir le milieu réactionnel sous forme liquide. La pression mise en oeuvre varie en fonction de la température du milieu réactionnel. Elle est généralement inférieure ou égale à 40 bar. De préférence, elle est inférieure ou égale à 35 bar. De manière particulièrement avantageuse, la pression est inférieure ou égale à 25 bar. En général, la pression est supérieure ou égale à 5 bar. De préférence, la pression est supérieure ou égale à 10 bar.

Le procédé de séparation selon l'invention peut être réalisé en discontinu
ou en continu.

Lorsque le procédé de séparation selon l'invention est réalisé en discontinu, la durée de la réaction entre le mélange hydrofluoroalcane/fluorure d'hydrogène et le composé organique varie en général de 10 min. à 5 h. De préférence, cette durée est d'au moins 0,5 h. De manière avantageuse, cette durée est d'au moins 1 h. En général, cette durée n'excède pas 4 h. De préférence, cette durée n'excède pas 2,5 h.

Lorsque le procédé de séparation selon l'invention est réalisé en continu, le temps de séjour des réactifs dans le réacteur est généralement d'au moins 0,5 h. Habituellement, il ne dépasse pas 30 h. Typiquement, il varie de 5 à 25 h. De préférence, il varie de 10 à 20 h. Par temps de séjour des réactifs dans le réacteur, on entend désigner le rapport entre le volume du milieu réactionnel et le débit volumique du milieu réactionnel à la sortie du réacteur.

Dans le cas où l'hydrofluoroalcane est le 1,1,1,3,3-pentafluorobutane et le composé organique est le 1,1,1,3,3-pentachlorobutane, de bons résultats sont obtenus avec un rapport molaire, entre le fluorure d'hydrogène et le 1,1,1,3,3-pentachlorobutane, inférieur à 15 mol/mol, de préférence, variant de 5 à 10 mol/mol, avec une température de réaction variant de 80 à 140°C, de préférence de 110 à 120°C, à une pression de 5 à 40 bar, de préférence de 15 à 25 bar, et avec un temps de séjour des réactifs dans le réacteur de 0,5 à 25 h.

La réaction entre le mélange hydrofluoroalcane/fluorure d'hydrogène et le composé organique dans le procédé selon l'invention peut être réalisée dans tout réacteur construit en un matériau résistant à la température, à la pression et aux réactifs utilisés, notamment au fluorure d'hydrogène.

De manière avantageuse, on élimine en continu tout ou partie du chlorure d'hydrogène formé par la réaction. En général, on élimine au moins 80 % du chlorure d'hydrogène.

Dans une variante du procédé selon l'invention, qui est préférée, on soumet le mélange de produits réactionnels obtenu dans la réaction du mélange hydrofluoroalcane/fluorure d'hydrogène avec au moins un composé organique capable de réagir avec du fluorure d'hydrogène à au moins une étape ultérieure de traitement destinée à récupérer l'hydrofluoroalcane. Des exemples d'étapes de traitement utilisables sont entre autres des traitements utilisables pour séparer de l'hydrofluoroalcane du fluorure d'hydrogène résiduel tels que par exemple une adsorption sur un solide, comme le NaF, un lavage à l'eau, une opération d'extraction, une séparation par une membrane appropriée, une distillation extractive ou au moins une distillation. Parmi ces traitements une distillation a donné de bons résultats.

Dans une variante on met en oeuvre une distillation en trois étapes. Une première étape de distillation (I) est destinée à récupérer au moins une fraction comprenant le fluorure d'hydrogène résiduel. Une deuxième étape de distillation (II) est destinée à récupérer au moins une fraction comprenant les impuretés non-volatiles. Une troisième étape de distillation (III) est destinée à récupérer au moins une fraction constituée de l'hydrofluoroalcane essentiellement pur. On peut adopter toute séquence de ces distillations donnant un résultat de séparation satisfaisant et permettant la récupération d'au moins une fraction contenant de l'hydrofluoroalcane essentiellement pur. Une séquence dans laquelle on effectue d'abord l'étape (I) ensuite l'étape (III) et ensuite l'étape (II) a donné de bons résultats.

Dans une forme spécifique de réalisation, l'invention concerne dès lors un procédé de séparation d'un mélange comprenant au moins un hydrofluoroalcane et du fluorure d'hydrogène, selon lequel on fait réagir ledit mélange avec au moins un précurseur chloré ou chlorofluoré de l'hydrofluoroalcane et ensuite, on récupère l'hydrofluoroalcane appauvri en fluorure d'hydrogène, de préférence en soumettant le mélange des produits réactionnels obtenu à au moins une distillation.

Dans la variante du procédé de séparation selon l'invention qui est effectuée en l'absence de catalyseur, le mélange réactionnel est avantageusement soutiré du réacteur sous forme liquide, puis soumis à au moins une étape de distillation.

La présente invention a également pour objet de fournir un procédé de préparation d'un hydrofluoroalcane, au départ d'un précurseur chloré ou chlorofluoré de l'hydrofluoroalcane et de fluorure d'hydrogène.

En conséquence, l'invention concerne un procédé de préparation d'un hydrofluoroalcane selon lequel on fait réagir, dans une première étape réactionnelle, au moins un précurseur chloré ou chlorofluoré de l'hydrofluoroalcane avec du fluorure d'hydrogène et on fait réagir, dans une étape réactionnelle ultérieure au moins une partie des produits formés lors de la première étape avec du fluorure d'hydrogène, une des étapes réactionnelles comprenant éventuellement un procédé de séparation selon l'invention.

Généralement, dans le procédé de préparation selon l'invention, de l'hydrofluoroalcane est formé dans la première étape réactionnelle et dans l'étape réactionnelle ultérieure. Souvent on maintient une conversion en hydrofluoroalcane de la somme des précurseurs chlorés ou chlorofluorés mis en oeuvre dans chaque étape d'au moins 5 % molaire. Plus souvent on maintient la conversion à au moins 10 % molaire. De préférence la conversion est maintenue à au moins 20 % molaire. De manière particulièrement préférée, la conversion est maintenue à au moins 50 % molaire.

De préférence, la conversion en hydrofluoroalcane de la somme des précurseurs chlorés ou chlorofluorés en hydrofluoroalcane mise en oeuvre dans chaque étape est différente dans la première étape réactionnelle et dans l'étape réactionnelle ultérieure. Dans ce cas, on maintient souvent une conversion comme décrit plus haut dans la première étape et on maintient une deuxième conversion plus élevée dans l'étape ultérieure. Souvent cette deuxième conversion de la somme des précurseurs chlorés ou chlorofluorés mis en oeuvre dans cette deuxième étape est d'au moins 70 % molaire. De préférence la deuxième conversion est d'au moins 90 % molaire.

Généralement, la teneur en fluorure d'hydrogène dans le milieu réactionnel est différente dans la première étape réactionnelle et dans l'étape ultérieure. De préférence, cette teneur est inférieure dans la première étape réactionnelle et supérieure dans l'étape ultérieure.

Généralement, la teneur en fluorure d'hydrogène dans le milieu réactionnel de la première étape réactionnelle est d'au moins 5 % en poids. De préférence la teneur est d'au moins 10 % en poids. Généralement, la teneur est d'au plus 20 % en poids. De préférence, elle est d'au plus 15 % en poids.

Généralement, la teneur en fluorure d'hydrogène dans le milieu réactionnel de l'étape réactionnelle ultérieure est d'au moins 40 % en poids. De préférence la teneur est d'au moins 60 % en poids. Généralement, la teneur est d'au plus 75 % en poids. De préférence, elle est d'au plus 70 % en poids.

Le procédé de préparation peut comprendre des étapes réactionnelles catalytiques et/ou des étapes réactionnelles effectuées en l'absence de catalyseur. Dans une variante, qui est préférée, le procédé de préparation selon l'invention comprend au moins une étape réactionnelle effectuée en l'absence de catalyseur et au moins une étape réactionnelle catalytique.

Par simplification, la suite de la description se rapporte uniquement à ce mode préféré du procédé de préparation selon l'invention, sans toutefois en limiter la portée à ce mode préféré.

Concernant l'étape réactionnelle effectuée en l'absence de catalyseur, les conditions réactionnelles préférées correspondent aux conditions décrites plus haut pour la réaction entre le mélange hydrofluoroalcane/fluorure d'hydrogène et le composé organique dans le procédé de séparation selon l'invention.

De préférence, les deux étapes réactionnelles sont effectuées en phase liquide et le mélange hydrofluoroalcane/fluorure d'hydrogène est soutiré de l'étape réactionnelle catalytique sous forme gazeuse, le plus souvent sous la forme d'une composition azéotropique.

Les hydrofluoroalcanes que l'on peut préparer par le procédé de préparation selon l'invention sont les mêmes que les hydrofluoroalcanes séparables de leurs mélanges avec le fluorure d'hydrogène par le procédé de séparation selon l'invention cités précédemment.

Les précurseurs chlorés ou chlorofluorés utilisables dans les étapes réactionnelles catalytique et non catalytique du procédé de préparation d'un hydrofluoroalcane selon l'invention sont les mêmes que les précurseurs chlorés
ou chlorofluorés utilisables dans le procédé de séparation selon l'invention cités précédemment. De préférence, le précurseur chloré ou chlorofluoré utilisé dans l'étape réactionnelle catalytique comprend un mélange d'intermédiaires chlorofluorés formés dans l'étape réactionnelle non catalytique par la réaction d'au moins une partie du fluorure d'hydrogène d'un mélange hydrofluoroalcane/fluorure d'hydrogène avec un précurseur chloré ou chlorofluoré de l'hydrofluoroalcane, l'étape réactionnelle non catalytique du procédé de préparation correspond alors à la réaction du procédé de séparation selon l'invention précédemment décrit.

Les différentes étapes de traitement utilisables dans le procédé de séparation selon l'invention s'appliquent également, le cas échéant, au présent procédé de préparation d'un hydrofluoroalcane.

Le procédé de préparation selon l'invention est particulièrement performant pour la préparation du 1,1,1,3,3-pentafluorobutane.

Lorsque le procédé de préparation selon l'invention est appliqué à la préparation du 1,1,1,3,3-pentafluorobutane, le précurseur de l'hydrofluoroalcane utilisé dans l'étape réactionnelle catalytique peut être du 1,1,1,3,3-pentachlorobutane, un mélange d'intermédiaires chlorofluorés, ou encore un mélange de ces produits avec du 1,1,1,3,3-pentachlorobutane. Les intermédiaires chlorofluorés formés dans l'étape réactionnelle non catalytique du procédé sont essentiellement des isomères des HCFC-363 et HCFC-364 c'est-à-dire du 1,1-dichloro-1,3,3-trifluorobutane (HCFC-363kfc), du 1,3-dichloro-1,1,3-trifluorobutane (HCFC-3631fb), du 3,3-dichloro-1,1,1-trifluorobutane (HCFC-363mfa), du 1-chloro-1,1,3,3-tétrafluorobutane (HCFC-3641fc) ou du 3-chloro-1,1,1,3-tétrafluorobutane (HCFC-364mfb) ou un mélange de ces composés.

D'autres intermédiaires éventuellement présents sont des (hydro)chloro(fluoro)butènes tels que, par exemple le 1,1-dichloro-1,3-difluorobut-2-ène et/ou le 1-chloro-1,1,3-trifluorobut-2-ène.

Lorsque le procédé de préparation selon l'invention est appliqué à la préparation du 1,1,1,3,3-pentafluorobutane, le précurseur de l'hydrofluoroalcane utilisé dans l'étape réactionnelle non catalytique du procédé est de préférence du 1,1,1,3,3-pentachlorobutane.

Comme catalyseur de la réaction de l'étape réactionnelle catalytique du procédé de préparation de l'hydrofluoroalcane selon l'invention, on peut mettre en oeuvre, par exemple les catalyseurs mentionnés plus haut comme catalyseurs utilisables dans le procédé de séparation selon l'invention.

La quantité de catalyseur mis en oeuvre dans l'étape réactionnelle catalytique du procédé de préparation d'un hydrofluoroalcane selon l'invention peut varier dans de larges limites. Elle est généralement d'au moins 0,5 % molaire. De préférence, elle est d'au moins 2 % molaire. De manière plus que préférée, elle est d'au moins 5 % molaire. Le plus souvent, elle ne dépasse pas 30 % molaire. De préférence, elle ne dépasse pas 20 % molaire. De manière plus que préférée, elle ne dépasse pas 10 % molaire. Dans l'étape réactionnelle catalytique, on met en oeuvre du fluorure d'hydrogène dans une quantité telle que le rapport molaire entre le fluorure d'hydrogène et le précurseur chloré ou chlorofluoré de l'hydrofluoroalcane est généralement d'au moins 3 mol/mol. De préférence, on travaille avec un rapport molaire d'au moins 5 mol/mol. Le plus souvent, ce rapport molaire ne dépasse pas 15 mol/mol. De préférence, il ne dépasse pas 10 mol/mol.

La température à laquelle on effectue la réaction de l'étape réactionnelle catalytique du procédé est généralement d'au moins 60°C. De préférence, elle est d'au moins 80°C. En général, elle ne dépasse pas 160°C. De préférence, elle ne dépasse pas 140°C.

La réaction entre le fluorure d'hydrogène et le précurseur chloré ou chlorofluoré de l'hydrofluoroalcane de l'étape réactionnelle catalytique du procédé est de préférence réalisée en phase liquide. Dans ce cas, la pression est choisie de manière à maintenir le milieu réactionnel sous forme liquide. La pression mise en oeuvre dans la réaction de l'étape réactionnelle catalytique du procédé varie en fonction de la température du milieu réactionnel. Elle est généralement inférieure ou égale à 35 bar. De manière particulièrement avantageuse, la pression est inférieure ou égale à 25 bar. En général, la pression est supérieure ou égale à 5 bar. De préférence, la pression est supérieure ou égale à 10 bar.

L'étape réactionnelle catalytique du procédé de préparation selon l'invention peut être réalisée en discontinu ou en continu.

Lorsque l'étape réactionnelle catalytique du procédé de préparation selon l'invention est réalisée en discontinu, la durée de la réaction varie en général de 10 min. à 5 h. De préférence, cette durée est d'au moins 0,5 h. De manière avantageuse, cette durée est d'au moins 1 h. En général, cette durée n'excède pas 4 h. De préférence, cette durée n'excède pas 2,5 h.

Lorsque l'étape réactionnelle catalytique du procédé de préparation selon l'invention est réalisée en continu, le temps de séjour des réactifs dans le réacteur est généralement d'au moins 0,5 h. Habituellement, il ne dépasse pas 50 h. Typiquement, il varie de 10 à 40 h. De préférence, il est de 10 à 30 h.

Lorsque le procédé de préparation selon l'invention est appliqué à la préparation du 1,1,1,3,3-pentafluorobutane, on effectue la réaction catalytique de préférence en présence de tétrachlorure de titane ou de tétrachlorure d'étain comme catalyseur, plus particulièrement en présence de tétrachlorure de titane. De bons résultats ont été obtenus à une température de 80 à 140°C, à une pression qui varie de 10 à 25 bar et avec un temps de séjour des réactifs dans le réacteur catalytique de 1 à 5 h.

La réaction de l'étape réactionnelle catalytique du procédé de préparation selon l'invention peut être réalisée dans tout réacteur construit en un matériau résistant à la température, à la pression et aux réactifs utilisés, notamment au fluorure d'hydrogène.

A la sortie de l'étape réactionnelle catalytique du procédé, on soutire, de préférence en phase gazeuse, un mélange de chlorure d'hydrogène et d'hydrofluoroalcane/fluorure d'hydrogène le cas échéant de composition azéotropique, mélange qui alimente l'étape réactionnelle non catalytique du procédé de préparation selon l'invention, éventuellement après séparation du chlorure d'hydrogène qu'il contient.

Une technique avantageuse consiste à effectuer la réaction catalytique dans un réacteur à l'ébullition à une température et une pression telle que le chlorure d'hydrogène et l'hydrofluoroalcane formés sont gazeux, alors que les réactifs et autres produits réactionnels sont essentiellement à l'état liquide. Le réacteur à l'ébullition est avantageusement surmonté d'une colonne de distillation afin de parfaire la séparation. Pour éviter l'accumulation des impuretés non volatiles dans le réacteur et maintenir l'activité du catalyseur, le réacteur de l'étape réactionnelle catalytique du procédé est avantageusement pourvu d'une purge.

Un mode de réalisation spécifique du procédé de préparation selon l'invention concerne un procédé de préparation d'un hydrofluoroalcane selon lequel, dans une étape réactionnelle catalytique, on fait réagir au moins un précurseur chloré ou chlorofluoré de l'hydrofluoroalcane avec du fluorure d'hydrogène en présence d'un catalyseur, et, dans une autre étape réactionnelle non catalytique, on fait réagir un mélange hydrofluoroalcane/fluorure d'hydrogène, provenant de l'étape réactionnelle catalytique, avec au moins un précurseur chloré ou chlorofluoré de l'hydrofluoroalcane selon le procédé de séparation selon l'invention décrit précédemment, au moins une partie du précurseur chloré ou chlorofluoré mis en oeuvre dans l'étape catalytique étant issu de l'étape non-catalytique.

Lorsque le catalyseur est un composé de titane, il s'est avéré possible de synthétiser l'hydrofluoroalcane en une seule étape réactionnelle.

Un troisième objet de l'invention concerne dès lors un procédé catalytique de fabrication d'un hydrofluoroalcane, utilisable à titre d'étape réactionnelle catalytique dans le procédé selon l'invention, dans lequel le catalyseur est un composé de titane. Souvent, dans le procédé catalytique selon l'invention la réaction est effectuée en phase liquide et le composé de titane est un halogénure de titane, de préférence le tétrachlorure de titane. Les conditions décrites ci-avant pour l'étape réactionnelle catalytique du procédé de préparation s'appliquent également au procédé catalytique selon l'invention. Le procédé catalytique selon l'invention convient bien pour la synthèse d'hydrofluoroalcanes comprenant de 3 à 6 atomes de carbone tels que par exemple le 1,1,1,3,3-pentafluoropropane et le 1,1,1,3,3-pentafluorobutane. Il convient particulièrement bien pour la synthèse de 1,1,1,3,3-pentafluorobutane, de préférence en une étape à partir du 1,1,1,3,3-pentachlorobutane.

Un quatrième objet de la présente invention concerne un procédé de synthèse d'un hydrofluoroalcane selon lequel on fait réagir en phase liquide au moins un précurseur chloré ou chlorofluoré de l'hydrofluoroalcane avec un rapport atomique F/Cl au sein du précurseur inférieur à 1 avec du fluorure d'hydrogène dans un milieu liquide dans lequel on maintient en permanence une teneur pondérale supérieure ou égale à 50 % en poids en composés organiques fluorés ou chlorofluorés présentant en moyenne un rapport atomique F/Cl d'au moins 1 de préférence d'au moins 1,2, de préférence particulière d'au moins 1,5. De préférence, ce procédé de synthèse est effectué au départ d'un précurseur chloré de l'hydrofluoroalcane en l'absence de catalyseur.

Aux fins de la présente invention, on entend en particulier désigner par composés organiques fluorés ou chlorofluorés, l'hydrofluoroalcane désiré et des intermédiaires chlorofluorés de l'hydrofluoroalcane. On entend également désigner éventuellement certains sous-produits réactionnels et/ou certaines impuretés amenées par le précurseur mis en oeuvre dans le procédé de synthèse. De préférence, la teneur pondérale en composés organiques fluorés
ou chlorofluorés dans le milieu liquide est au moins égale à 70 %. De manière particulièrement préférée, elle est au moins égale à 80 %. En général, elle ne dépasse pas 99 % en poids. De préférence, elle ne dépasse pas 98,5 % en poids.

Les conditions opératoires de la réaction décrite dans le procédé de séparation selon l'invention sont directement applicables au présent procédé de synthèse d'un hydrofluoroalcane. Lorsque l'on fait suivre le présent procédé de synthèse d'un hydrofluoroalcane par les étapes de distillation décrites dans le procédé de séparation selon l'invention, les conditions opératoires sont de préférence telles que l'on soutire en phase liquide un milieu réactionnel qui contient moins de fluorure d'hydrogène que dans le mélange azéotropique hydrofluoroalcane/fluorure d'hydrogène.

Ce procédé s'applique avantageusement à la synthèse du 1,1,1,3,3-pentafluorobutane au départ de 1,1,1,3,3-pentachlorobutane.

Lorsque le procédé est appliqué à la synthèse du 1,1,1,3,3-pentafluorobutane, de préférence, au moins 50 % du milieu liquide réactionnel est constitué de 1,1,1,3,3-pentafluorobutane et des isomères des HCFC-363 et HCFC-364.

Ce procédé de synthèse est avantageux car la présence de l'hydrofluoroalcane désiré et de ses intermédiaires chlorofluorés dans le milieu liquide réactionnel en proportions importantes a un effet de solvant sur le mélange du fluorure d'hydrogène et du précurseur chloré ou chlorofluoré de l'hydrofluoroalcane, qui permet d'augmenter la productivité de la réaction, en particulier parce que certains intermédiaires chlorofluorés peu réactifs, qui s'accumulent dans le milieu en l'absence de l'hydrofluoroalcane semblent être formés en quantités beaucoup plus limitées dans les conditions du procédé de synthèse selon l'invention.

L'invention concerne aussi des compositions azéotropiques ou pseudo-azéotropiques constituées essentiellement de 1,5 % molaire à 27,5 % molaire de 1,1,1,3,3-pentafluorobutane et de 72,5 % molaire à 98,5 % molaire de fluorure d'hydrogène.

Fondamentalement, l'état thermodynamique d'un fluide est défini par quatre variables interdépendantes : la pression (P), la température (T), la composition de la phase liquide (X) et la composition de la phase gazeuse (Y). Un azéotrope vrai est un système particulier à 2 ou plusieurs composants pour lequel, à une température donnée et à une pression donnée, la composition de la phase liquide X est exactement égale à la composition de la phase gazeuse Y. Un pseudo-azéotrope est un système à 2 ou plusieurs composants pour lequel, à une température donnée et à une pression donnée, X est substantiellement égal à Y. En pratique, cela signifie que les constituants de tels systèmes azéotropiques et pseudo-azéotropiques ne peuvent pas être séparés facilement par distillation.

Aux fins de la présente invention, on entend par mélange pseudo-azéotropique, un mélange de deux constituants dont le point d'ébullition (à une pression donnée) diffère du point d'ébullition de l'azéotrope vrai de 0,5°C au maximum. Les mélanges dont le point d'ébullition diffère du point d'ébullition de l'azéotrope vrai de 0,2°C au maximum sont préférés. Les mélanges dont le point d'ébullition diffère du point d'ébullition de l'azéotrope vrai de 0,1 °C au maximum sont particulièrement préférés.

Le 1,1,1,3,3-pentafluorobutane et le fluorure d'hydrogène forment un azéotrope ou un pseudo-azéotrope binaire lorsque leur mélange contient environ de 72.5 à 98.5 % molaire de fluorure d'hydrogène. Sous une pression de 1 bar, la composition binaire est constituée essentiellement d'environ 91 à 98,5 % molaire de fluorure d'hydrogène et 1,5 à 9 % molaire de 1,1,1,3,3-pentafluorobutane et présente un point d'ébullition minimum d'environ 18°C. Sous une pression de 10 bar, la composition binaire est constituée essentiellement de 78 à 85 % molaire de fluorure d'hydrogène et 15 à 22 % molaire de 1,1,1,3,3-pentafluorobutane et présente un point d'ébullition minimum d'environ 90°C. Sous une pression de 12 bar, la composition binaire est constituée essentiellement d'environ 75 à 84 % molaire de fluorure d'hydrogène et 16 à 25 % molaire de 1,1,1,3,3-pentafluorobutane et présente un point d'ébullition minimum d'environ 97°C.

L'évolution marquée de la concentration des constituants de l'azéotrope avec la pression est tout à fait inattendue. Il est ainsi possible en utilisant le procédé selon l'invention d'isoler les constituants essentiellement purs de l'azéotrope ainsi que des fractions azéotropiques à une pression donnée, enrichies soit en fluorure d'hydrogène soit en 1,1,1,3,3-pentafluorobutane.

Les compositions selon l'invention peuvent être utilisées, par exemple, pour l'épuration de 1,1,1,3,3-pentafluorobutane. Lorsqu'on veut épurer du 1,1,1,3,3-pentafluorobutane contenant des impuretés qui ne forment pas d'azéotrope avec le fluorure d'hydrogène ou dont l'azéotrope avec le fluorure d'hydrogène présente un point d'ébullition nettement différent des compositions selon l'invention, on peut utiliser les compositions pour séparer le 1,1,1,3,3-pentafluorobutane et le fluorure d'hydrogène d'une part et les impuretés d'autre part. Un exemple d'une telle utilisation est la séparation de 1,1,1,3,3-pentafluorobutane et de fluorure d'hydrogène d'un mélange réactionnel obtenu dans un procédé de synthèse de 1,1,1,3,3-pentafluorobutane par hydrofluoration d'un précurseur chloré tel que, par exemple, le procédé mentionné plus haut. Dans cette utilisation, on préfère mettre en oeuvre une composition selon l'invention riche en 1,1,1,3,3-pentafluorobutane. Généralement la composition contient au moins 10 % molaire de 1,1,1,3,3-pentafluorobutane. Plus souvent, la composition contient au moins 15 % molaire de 1,1,1,3,3-pentafluorobutane. De préférence elle contient au moins 20 % molaire de 1,1,1,3,3-pentafluorobutane.

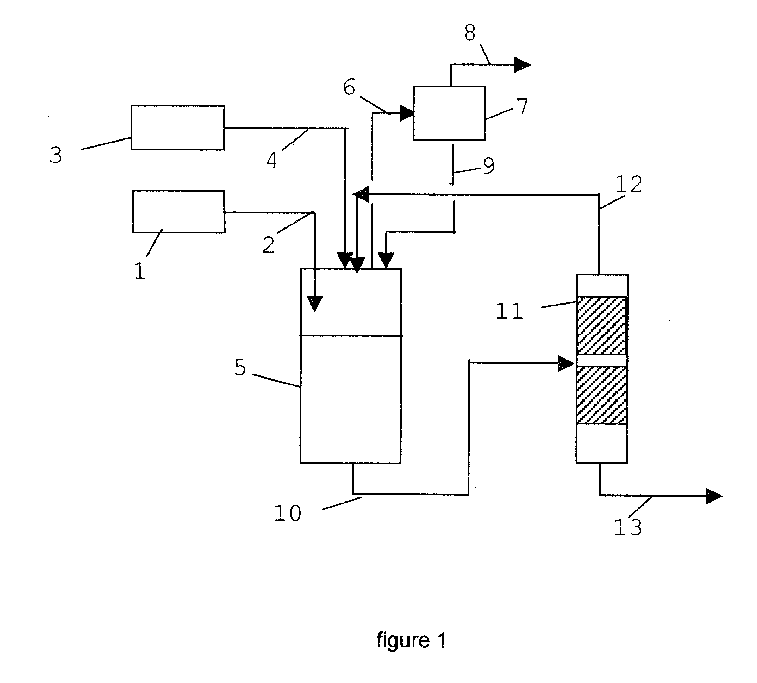

Les figures 1 à 4 illustrent respectivement des modes de réalisation spécifiques du procédé de séparation d'un mélange comprenant au moins un hydrofluoroalcane et du fluorure d'hydrogène, du procédé de fabrication d'un hydrofluoroalcane et les compositions azéotropiques ou pseudo-azéotropiques conformes à la présente invention.

La Figure 1 illustre la variante du procédé de séparation selon l'invention dans laquelle la réaction entre le mélange hydrofluoroalcane/fluorure d'hydrogène et le composé organique, en l'occurrence un précurseur chloré ou chlorofluoré de l'hydrofluoroalcane, est effectuée en l'absence de catalyseur.

Dans l'installation telle que schématisée à la figure 1, le précurseur chloré
ou chlorofluoré de l'hydrofluoroalcane provenant d'un réservoir (1) est introduit via une voie (2) dans un réacteur (5). Le mélange hydrofluoroalcane/fluorure d'hydrogène à séparer selon l'invention provenant d'un réservoir (3) est introduit via une voie (4) dans le réacteur (5). Dans le réacteur (5), le fluorure d'hydrogène du mélange hydrofluoroalcane/fluorure d'hydrogène réagit avec le précurseur chloré ou chlorofluoré de l'hydrofluoroalcane pour donner un mélange de produits réactionnels contenant de l'hydrofluoroalcane, du fluorure d'hydrogène en quantité réduite, du chlorure d'hydrogène, des intermédiaires chlorofluorés, éventuellement du précurseur chloré ou chlorofluoré non réagi et des impuretés non volatiles. Le chlorure d'hydrogène est soutiré sous forme gazeuse du mélange des autres produits réactionnels via la voie (6), séparé des autres produits réactionnels éventuellement entraînés dans le séparateur (7), et soutiré par la voie (8) Les autres produits réactionnels éventuellement entraînés retournent au réacteur (5) via la voie (9).

Le mélange des autres produits réactionnels est envoyé sous forme liquide, via la voie (10), dans une étape de distillation (11). En tête de la distillation (12), on recueille un mélange comprenant de l'hydrofluoroalcane et de fluorure d'hydrogène qui est éventuellement recyclé au réacteur (5) via la voie (12). En pied de la distillation (13), on recueille un mélange de produits contenant principalement de l'hydrofluoroalcane, des intermédiaires chlorofluorés de l'hydrofluoroalcane, éventuellement du précurseur non réagi et des impuretés non volatiles.

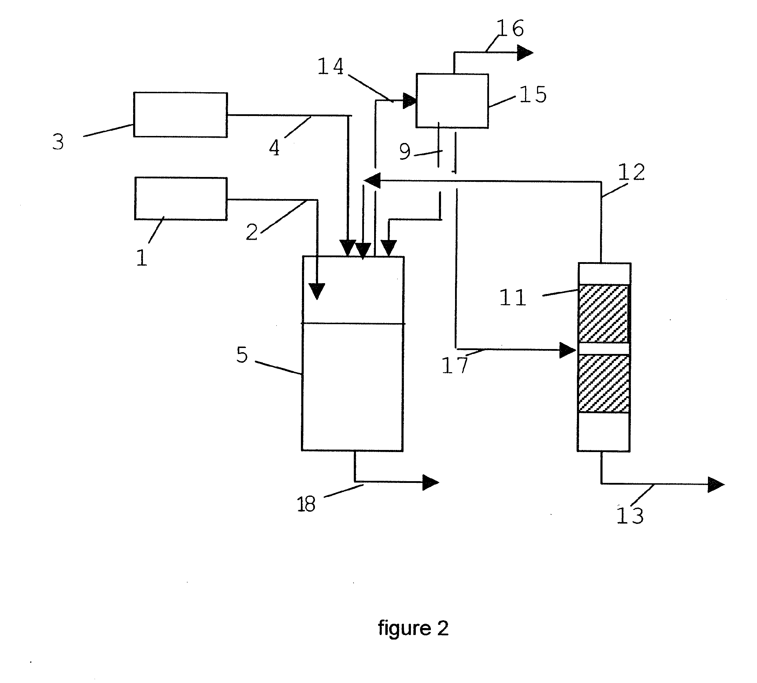

La Figure 2 illustre la variante du procédé de séparation selon l'invention dans laquelle la réaction entre le mélange hydrofluoroalcane/fluorure d'hydrogène et le composé organique, en l'occurrence un précurseur chloré ou chlorofluoré de l'hydrofluoroalcane, est effectuée en présence de catalyseur. Les parties de cette installation qui sont identiques à celles de l'installation décrite à la figure 1 portent les mêmes numéros de référence. Ces parties ne seront pas décrites à nouveau.

Dans cette variante, les produits réactionnels de la réaction sont soutirés, sous forme gazeuse via la voie (14), et envoyés dans le séparateur (15). Le chlorure d'hydrogène est soutiré par la voie (16) Les autres produits réactionnels sont envoyés, via la voie (17), dans une étape de distillation (11). En tête de la distillation (12), on recueille un mélange comprenant de l'hydrofluoroalcane et de fluorure d'hydrogène qui est éventuellement recyclé au réacteur (5) via la voie (12). En pied de la distillation (13), on recueille un mélange de produits contenant principalement de l'hydrofluoroalcane, des intermédiaires chlorofluorés de l'hydrofluoroalcane, éventuellement du précurseur non réagi et des impuretés non volatiles.

Pour éviter l'accumulation des impuretés non volatiles dans le réacteur (5) et maintenir l'activité du catalyseur, le réacteur (5) est pourvu d'une purge (18).

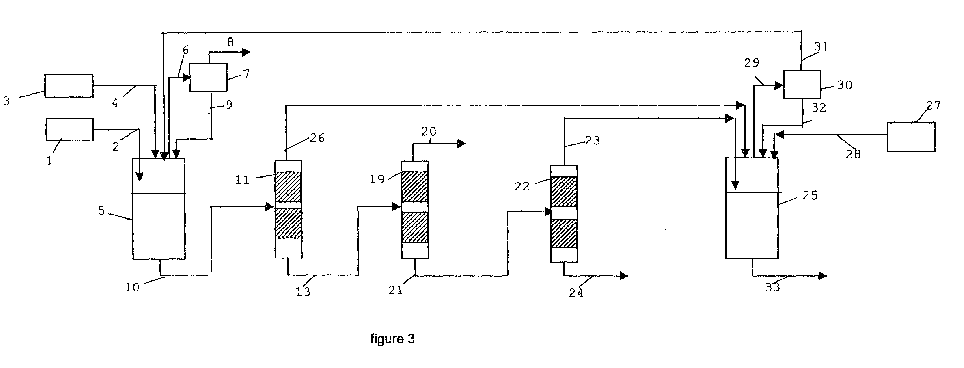

Le procédé de préparation de l'hydrofluoroalcane en deux étapes réactionnelles selon l'invention est illustré par le schéma réactionnel donné à la figure 3.

Les parties de cette installation qui sont identiques à celles de l'installation décrite à la figure 1 portent les mêmes numéros de référence. Ces parties ne seront pas décrites à nouveau.

Le mélange de produits contenant principalement de l'hydrofluoroalcane, des intermédiaires chlorofluorés de l'hydrofluoroalcane, éventuellement du précurseur non réagi et des impuretés non volatiles recueilli par la voie (13) est introduit dans la distillation (19). En tête de la distillation (20), on recueille l'hydrofluoroalcane essentiellement pur. En pied de la distillation (21), on recueille un mélange comprenant de l'hydrofluoroalcane, des intermédiaires chlorofluorés de l'hydrofluoroalcane, des impuretés non volatiles et éventuellement du précurseur non réagi, qui est envoyé dans une troisième distillation (22).

En tête de la distillation (23), on recueille un mélange comprenant de l'hydrofluoroalcane, des intermédiaires chlorofluorés de l'hydrofluoroalcane, et éventuellement du précurseur non réagi. En pied de la distillation (24), on recueille les impuretés non volatiles qui sont éliminées de l'installation.

Le mélange de produits recueilli en tête de la distillation (22) est envoyé dans un réacteur catalytique (25) via la voie (23). En outre, à la différence de l'installation décrite à la figure 1, le mélange d'hydrofluoroalcane et de fluorure d'hydrogène recueilli en tête de la colonne (11) est également envoyé au réacteur (25) via la voie (26).

Le réacteur (25), qui contient un catalyseur, est alimenté via la voie (28) en fluorure d'hydrogène provenant du réservoir (27). Dans le réacteur (25), le fluorure d'hydrogène réagit avec les produits provenant de la colonne (22). Un mélange hydrofluoroalcane/-fluorure d'hydrogène et de chlorure d'hydrogène est soutiré du réacteur (25) sous forme gazeuse via la voie (29) et introduit dans un séparateur (30). Le mélange hydrofluoroalcane/fluorure d'hydrogène et de chlorure d'hydrogène est envoyé via la voie (31) au réacteur (5). Les autres produits réactionnels retournent au réacteur (25) via la voie (32).

Pour éviter l'accumulation des impuretés non volatiles dans le réacteur et maintenir l'activité du catalyseur, le réacteur (25) est pourvu d'une purge (33).

Les exemples qui suivent sont destinés à illustrer la présente invention sans toutefois en limiter la portée.

Dans l'exemple 1 ci-dessous, le taux de transformation (TT) du 1,1,1,3,3-pentachlorobutane (PCBa) est le rapport, exprimé en pour-cent, entre la quantité mise en oeuvre diminuée de la quantité non convertie au terme de la réaction et la quantité mise en oeuvre.

### Exemple 1

L'exemple 1 a été mené dans une installation conforme à la figure 1.

Dans un réacteur en acier inoxydable de 0,5 1, équipé d'un agitateur mécanique à pales, d'une sonde de température et d'un tube plongeant permettant d'effectuer des prélèvements en phase liquide en cours d'essai, on a introduit 5,02 moles de fluorure d'hydrogène sous la forme d'un mélange de composition azéotropique contenant 0,475 mole 1,1,1,3,3-pentafluorobutane (rapport molaire HF/1,1,1,3,3-pentafluorobutane de 10,6 mol/mol) et 0,739 mole de 1,1,1,3,3-pentachlorobutane. Le réacteur a ensuite été plongé dans un bain thermostatisé, maintenu à une température de 120°C sous agitation continue. La pression a été régulée à 25 bar. Des prélèvements ont été réalisés après 1 h, 3 h et 30 h de réaction. Les résultats sont repris dans le Tableau ci-dessous. Le taux de transformation du 1,1,1,3,3-pentachlorobutane mis en oeuvre a varié de 94 à 100 % molaire, respectivement, entre 1 h et 30 h de réaction. Le rapport molaire HF/1,1,1,3,3-pentafluorobutane s'est abaissé de 6 à 1,8 mol/mol, respectivement, entre 1 h et 30 h de réaction. Les quantités de 1,1,1,3,3-pentafluorobutane (HFC-365mfc) et des intermédiaires chlorofluorés (HCFC-361, -362, -363 et -364) présents dans le milieu réactionnel après les différents prélèvements sont également indiqués.

**Tableau**

| Durée de réaction, h | 1 | 3 | 30 |
|---|---|---|---|
| TT PCBa, % | 94 | > 99,9 | 100 |
| Quantité de HFC-365mfc, mol (dans le milieu réactionnel) | 0,51 | 0,64 | 0,99 |
| Quantité des HCFC-364, mol (dans le milieu réactionnel) | 0,30 | 0,35 | 0,074 |
| Quantité des HCFC-363, mol (dans le milieu réactionnel) | 0,09 | 0,13 | 0,10 |
| Quantité des HCFC-362, mol (dans le milieu réactionnel) | 0,16 | 0,04 | < 0,007 |
| Quantité des HCFC-361, mol (dans le milieu réactionnel) | 0,007 | < 0,007 | < 0,007 |
| HF consommé, mol | 1,94 | 2,70 | 3,20 |
| HF restant, mol | 3,08 | 2,30 | 1,80 |
| HF/HFC-365mfc, mol/mol | 6,0 | 3,6 | 1,8 |

### Exemple 2

Dans le même réacteur qu'à l'exemple 1, on a introduit 5,0 moles de fluorure d'hydrogène et 0,053 mole de tétrachlorure de titane. Le réacteur a ensuite été plongé dans un bain thermostatisé et maintenu à une température de 135°C sous agitation continue. La pression a été régulée à 25 bar. On a alimenté en continu le réacteur avec 0,1mol/h de 1,1,1,3,3-pentachlorobutane et 1mol/h de fluorure d'hydrogène. Le réacteur a été dégazé en continu de façon a maintenir le niveau du mélange réactionnel dans le réacteur essentiellement constant. La quantité de 1,1,1,3,3 pentafluorobutane dans les gaz soutirés correspondait à un rendement par rapport à la quantité de 1,1,1,3,3-pentachlorobutane mise en oeuvre de 97 %.

On a effectué la réaction pendant 30 heures dans ces conditions et le rendement a été stable.

Les gaz soutirés ont été un mélange de fluorure d'hydrogène/1,1,1,3,3-pentafluorobutane dans un rapport molaire proche de 5/1 pouvant avantageusement être mis en oeuvre dans le procédé de séparation selon l'invention.

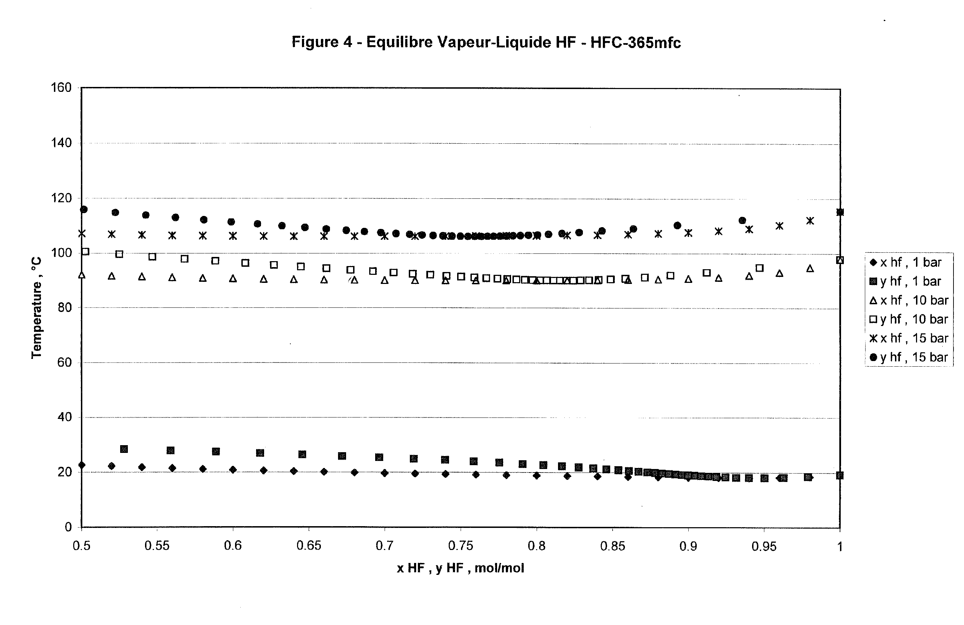

Les exemples 3 à 5 dans la figure 4, représentent les équilibres vapeur/liquide de compositions binaires de 1,1,1,3,3-pentafluorobutane et de fluorure d'hydrogène à trois différentes pressions. x HF signifie la proportion molaire de fluorure d'hydrogène dans la phase liquide et y HF signifie la proportion molaire de fluorure d'hydrogène dans la phase gazeuse. Les données sur lesquelles sont basées les courbes ont été obtenues par calcul à l'aide du logiciel Aspen Plus® de la société Aspen Tech® à partir de propriétés thermodynamiques mesurées et calculées de mélanges de 1,1,1,3,3-pentafluorobutane et de fluorure d'hydrogène. L'exemple 3 est donné par les courbes correspondant à une pression de 1 bar. L'exemple 4 est donné par les courbes correspondant à une pression de 10 bar. L'exemple 5 est donné par les courbes correspondant à une pression de 15 bar.

## Revendications

1. Compositions azéotropiques ou pseudo-azéotropiques constituées essentiellement de 1,5 % molaire à 27,5 % molaire de 1,1,1,3,3-pentafluorobutane et de 72,5 % molaire à 98,5 % molaire de fluorure d'hydrogène.

2. Mélange azéotropique conforme aux compositions azéotropiques ou pseudo-azéotropiques selon la revendication 1 contenant du fluorure d'hydrogène et du 1,1,1,3,3-pentafluorobutane dans un rapport molaire fluorure d'hydrogène /1,1,1,3,3-pentafluorobutane d'environ 11 mol/mol à une pression de 3 bar.

3. Composition azéotropique binaire selon revendication 1 constituée essentiellement d'environ 91 à 98,5 % molaire de fluorure d'hydrogène et 1,5 à 9 % molaire de 1,1,1,3,3-pentafluorobutane sous une pression de 1 bar, présentent une point d'ébullition d'environ 18°C.

4. Utilisation de compositions azéotropiques ou pseudo-azéotropiques selon l'une quelconque des revendications 1 à 3 pour l'épuration de 1,1,1,3,3-pentafluorobutane.

5. Utilisation selon revendication 4 pour l'épuration de 1,1,1,3,3-pentafluorobutane contenant des impuretés qui ne forment pas d'azéotrope avec le fluorure d'hydrogène ou dont l'azéotrope avec le fluorure d'hydrogène présente un point d'ébullition nettement différent des compositions azéotropes ou pseudo-azéotropiques constituées essentiellement de 1,5 % molaire à 27,5 % molaire de 1,1,1,3,3-pentafluorobutane et de 72,5 % molaire à 98,5 % molaire de fluorure d'hydrogène.

6. Utilisation de compositions azéotropiques ou pseudo-azéotropiques selon l'une quelconque des revendications 1 à 3 pour la préparation de 1,1,1,3,3-pentafluorobutane.

7. Utilisation selon revendication 6 pour la préparation de 1,1,1,3,3-pentafluorobutane comprenant la réaction d'un précurseur chloré ou chlorofluoré du 1,1,1,3,3-pentafluorobutane avec les compositions azéotropiques ou pseudo-azéotropiques.

8. Procédé de synthèse d'un hydrofluoroalcane selon lequel on fait réagir en phase liquide au moins un précurseur chloré ou chlorofluoré de l'hydrofluoroalcane avec un rapport atomique F/Cl inférieur à 1 avec du fluorure d'hydrogène dans un milieu liquide dans lequel on maintient en permanence une teneur pondérale supérieure ou égale à 50 % en poids en composés organiques fluorés ou chlorofluorés présentant en moyenne un rapport atomique F/Cl d'au moins 1 de préférence d'au moins 1,2, de préférence particulière d'au moins 1,5.

9. Procédé catalytique de fabrication d'un hydrofluoroalcane comprenant 3 à 6 atomes de carbone selon lequel on fait réagir au moins un précurseur chloré ou chlorofluoré de l'hydrofluoroalcane avec du fluorure d'hydrogène dans lequel le catalysateur est un composé de titane.

10. Procédé selon la revendication 9 dans lequel l'hydrofluoroalcane est synthétisé en une seule étape réactionnelle.

11. Procédé selon la revendication 9 dans lequel la réaction est effectuée en phase liquide et le composé de titane est un halogénure de titane, de préférence le tetrachlorure de titane.

12. Procédé selon la revendication 9 dans lequel l'hydrofluoroalcane est 1,1,1,3,3-pentafluoropropane ou 1,1,1,3,3-pentafluorobutane.

13. Procédé selon la revendication 12 selon 1,1,1,3,3-pentafluorobutane est synthétisé en une étape à partir du 1,1,1,3,3-pentachlorobutane.
